# EUROPEAN PATENT APPLICATION

(11) **EP 2 289 519 A2**
(43) Date of publication of application: **02.03.2011**
(21) Application number: 10011519.5
(22) Date of filing: 18.02.2003
(51) Int. Cl.: A61K 31/55, A61P 25/28

(54) **Use of modulators of nicotinic receptors for treatment of cognitive dysfunction**

(30) Priority: 22.02.2002 US 358917 P; 14.03.2002 US 99858
(62) Divisional of application: 03711042.6
(71) Applicant: Davis, Bonnie M., Syosset, NY 11791 (US)
(72) Inventor: Davis, Bonnie M., Syosset, NY 11791 (US)
(74) Representative: Richards, John

(57) **Abstract**

A method for treating the effects of low LDL-cholesterol values in the brain on cognitive performance or other central nervous system functions by modulating nicotinic receptors by administering an effective amount of a nicotinic allosteric potentiator, an acetylcholineseterase inhibitor, nicotine, a nicotinic agonist or a mixture thereof to a patient in need of such modulation.

## Description

The present invention relates to use of nicotinic receptor modulators, including galanthamine and lycoramine and their analogs for treatment of cognitive and other central nervous system dysfunction resulting from low LDL-cholesterol values, for example resulting from use of drugs for control of cholesterol and in particular LDL-cholesterol.

Muldoon et al. (Am J Med 108, 538 May 2000)reviewed reports that hypercholesterolemia has been associated with better scores on some tests of cognitive function in humans and that reducing the cholesterol content of neural tissue in laboratory animals affects pain threshold and learning behaviors. Furthermore calorie restricted diets which lower cholesterol in humans may "slightly impair mental efficiency". They also reported that at LDL-cholesterol levels below 109 mg/dl there was a highly significant decrease in cognitive function (p=.007). In humans, Kostis et al. in J Clin Pharmacol 34(10) 989 (1994)refer to reports of performance deficits being attributed to the statin class of drugs which inhibit synthesis of cholesterol. However, their own work showed no effect on cognitive performance. Roth et al (Clin Cardiol 15(6) 426-432 (1992)) describe a three week study comparing lovastatin to simvastatin in 22 young normal volunteers, showing impairment by lovastatin. However, other studies showed no such impairment (Gengo et al. in Clin Cardiol 18(4): 209-214 (1995)), Cutler et al in Br J Clin Pharmacol 39(3): 333 - 336 (1995), Harrison in Br J Clin Pharmacol 37(3): 231 -236 (1994 and Gibellato et al. in Aviat. Space Environ Med 72(9), 805 -12 (2001)). NCEP III guidelines recommend LDL-cholesterol levels of less than 100mg/dl for 32 million Americans including 39% of the elderly. (Fedder et al. Circulation 105(2): 152-6 (2002).

Muldoon states that "the mechanism by which lovastatin might affect cognitive function is unknown". However, I believe that the effect is probably due to impairment of nicotinic receptor function as a result of the presence of insufficient amounts of cholesterol.

Burger et al. in a review in Cell Mol Life Sci 57:1577-1592 (2000) note that it is known that cholesterol may stabilize receptors in defined conformations related to their biological functions and discuss the functional dependence of nicotinic acetylcholine receptors on the presence of cholesterol.

Nicotinic receptors are known to be ligand-gated ion channels allowing passage of cations into a cell. Schrattenholz et al Molecular Pharmacology 49: 1-6 (1996) note that cationic translocation through the nicotinic receptor is stimulated by naturally occurring acetyl choline and can be enhanced by allosteric modulation which can prolong and enhance the opening of the gate and result in increased depolarization without desensitization. It is noted that galanthamine may act to prolong the opening of such channels.

Lloyd et al. in J. Pharmacology and Experimental Therapeutics 292(2): 461-467 (2000) note that there is considerable molecular diversity in the subunits of nicotinic acteylcholine receptors and describes a number of compounds which interact with them.

In my prior application PCT Publication WO/0143697 I have described the modulation of nicotinic receptors as being useful in improving attentional functions, relieving pain, treating nicotine and similar addictions, treating anxiety and depression, treating and retarding the progression of Alzheimer's and Parkinson's diseases, neuroprotection against neurodegenerative disorders, alcohol, glutamate and other toxic effects and treatment of schizophrenia.

Galanthamine is an alkaloid isolated initially from galanthus nivalis, the snowdrop, which has been used for many years as an acetylcholinesterase inhibitor. The principal use in humans has been the postoperative reversal of neuromuscular blockade but in recent years it has started to be used for the treatment of Alzheimer's Disease.

The present invention provides a method for treating the effects of low LDL-cholesterol values in the brain, for example caused by HMG-CoA reductase inhibitors (statins) on cognitive performance by modulating nicotinic receptors by administering an effective amount of a nicotinic allosteric potentiator, an acetylcholinesterase inhibitor, nicotine or a nicotinic agonist to a patient in need of such modulation.

Suitable compounds for modulating nicotinic receptors for the purposes of the present invention include galanthamine, lycoramine, analogs of either of them which have nicotinic allosteric potentiating properties, donepezil and rivastigmine. Other compounds which may be of use include nicotine itself (for example administered by a patch), other nicotinic agonists, and potentiators of nicotinic receptors.

Analogs of galanthamine that are of use in the present invention are those having good nicotinic properties. Classical neurochemical techniques, such as employed by Kostowski and Gomulka (op cit) may be used to identify compounds with nicotinic properties. In these, an outcome measure known to be cholinergic, such as an electrical potential or other biological function, is blocked with a nondepolarizing agent such as hexamethonium. Newer techniques, such as patch-clamp recordings in hippocampal slices (Alkondon M, Pereira EF, Eisenberg HM, Albuquerque EX, Choline and selective agonists identify two subtypes of nicotinic acetylcholine receptors that modulate GABA release from CA1 interneurons in rat hippocampal slices. J Neurosci 19(7):2693-2705, 1999), current and voltage clamp modes, (Frazier CJ, Rollins YD, Breese CR, Leonard S, Freedman R, Dunwiddie TV (Acetylcholine activates an alpha-bungarotoxin-sensitive nicotinic current in rat hippocampal intemeurons, but not pyramidal cells. J Neurosci 18(4)1187-1195, 1998) or electrophysiological recordings (Stevens KE, Kem WR, Freedman R, Selective alpha 7 nicotinic receptor stimulation normalizes chronic cocaine-induced loss of hippocampal sensory inhibition of C3H mice. Biol Psychiatry 46(10)1443-50, 1999), or techniques such as employed by Storch et al, above, may be also be used to identify compounds which are candidates for appropriate safety, pharmacokinetic and finally studies in humans. In addition, pharmacological reversal trials with nicotinic receptor inhibitors such as hexamethonium mecamylamine, methylyaconitine dihydro beta erythroidine may be used to identify nicotinic mechanisms.

Such compounds include analogs wherein at least one of the methoxy, hydroxy or methyl groups of galanthamine or lycoramine is replaced as follows:
the methoxy group by another alkoxy group of from one to six carbon atoms, a hydroxy group, hydrogen, an alkanoyloxy group, a benzoyloxy or substituted benzoyloxy group, a carbonate group or a carbamate group or a trialkylsilyloxy group;
the hydroxy group by an alkoxy group of from one to six carbon atoms, hydrogen, an alkanoyloxy group, a benzoyloxy or substituted benzoyloxy group, a carbonate group or a carbamate group;
the N-methyl group by hydrogen, alkyl, benzyl, cyclopropylmethyl group or a substituted or unsubstituted benzoyloxy group.

Other suitable analogs may be found for example in International Patent Publication WO 88/08708 and an article by Bores and Kosley in Drugs of the Future 21:621-631 (1996).

Alkanoyloxy, carbamate and carbonate groups of use in the compounds of the present invention typically contain up to ten carbon atoms. The substituent groups, are typically selected from alkyl or alkoxy groups of from 1 to 6 carbon atoms, halo groups, and haloalkyl groups such as trifluoromethyl. When reference is made to alkyl groups, where the context permits, the term also include groups which are or contain cycloalkyl groups including adamantyl groups. Aryl groups are typically phenyl or naphthyl but may include heteroaryl such as morpholino. The carbamate groups may be mono or di-substituted and in the case of disubstituted carbamates, each of the groups may be as just specified. For example a dimethyl carbamate group may be used.

Galanthamine has the following structure:

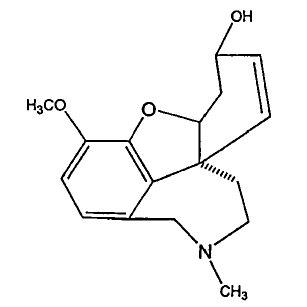

Lycoramine is similar but has only a single bond between the 3 and 4 positions.

Particularly useful analogs of galanthamine and lycoramine for use in the present invention include analogs thereof wherein the hydroxy and/or methoxy groups are replaced by carbamate groups, for example 2-n-butyl carbamates.

Other compounds that may be of use are those wherein the methoxy group of galanthamine or lycoramine is replaced by a hydrogen, hydroxy or alkoxy group of from two to six carbon atoms or an acyloxy group, for example an alkanoyloxy or benzoyl group, of from one to seven carbon atoms, more preferably of two to seven carbon atoms. Other compounds that may be of interest are those wherein the methoxy group is replaced by a mono or dialkyl carbamate or carbonate group wherein the alkyl groups contain from 1 to 8 carbon atoms, preferably of from 4 to 6 carbon atoms or wherein the methoxy group thereof is replaced by an aryl carbamate or carbonate group wherein said aryl group is selected from phenyl, naphthyl, substituted phenyl and substituted naphthyl groups wherein said substituent is selected from alkyl and alkoxy groups of from 1 to 6 carbon atoms, trifluoro methyl groups and halo groups. Care should, however, be taken with such 13-carbamates to ensure that there are no toxicity problems with the intended method of use.

Other useful analogs include compounds wherein, independently of whether or not the methoxy group has been replaced, the hydroxy group is replaced by an alkoxy group of from one to six carbon atoms, hydrogen, an acyloxy group, for example an alkanoyloxy group, typically of from 1 to 7 carbon atoms, a benzoyloxy or substituted benzolyloxy group wherein said substituent is selected from alkyl and alkoxy groups of from 1 to 6 carbon atoms, trifluoro methyl groups and halo groups, or a carbonate group, preferably or a carbamate group which may be a mono or dialkyl or an aryl carbamate or carbonate wherein the alkyl groups contain from 1 to 8 carbon atoms, preferably of from 4 to 6 carbon atoms or said aryl group is selected from phenyl, naphthyl, substituted phenyl and substituted naphthyl groups wherein said substituent is selected from alkyl and alkoxy groups of from 1 to 6 carbon atoms, trifluoro methyl groups and halo groups.

Determination of the suitability of galanthamine or lycoramine analogs may be made by determination of the increase in current generated in PC 12 cells exposed to low doses of acetyl choline (Schrattenholz, et al Molecular Pharmacology 49: 1-6 1996) or by reversal of cognitive enhancement by the nicotinic blocker, mecamylamine (Zarrindast et al in Eur J Pharmacol 295(1):1-6 (1996), Matsuyama et al. in Eur J Neurosci 12(10):3741-7 (2000), Levin et al. in Behav Pharmacol 4(2): 179-182 (1993), Lozano et al in J Comp Physiol [A] 187(4):249-54 (2001), Bettany in Pharmacol Biochem Behav 70(4): 467-74 (2001).

Although nicotinic allosteric potentiators are the preferred compounds for use in the present invention, other useful compounds may include nicotinic agonists such as those described in Lloyd et al. in J. Pharmacology and Experimental Therapeutics 292(2): 461-467 (2000) where there are listed GTS-21, ABT-418, SIB,1508Y, RJR 2403, ABT 594, SIB 1553A, DBO 83, AR-R 17779 and ABT-089.

A number of patents assigned to the University of Florida describe the use of anabaseine, its derivatives and anabasine as being of use as nicotinic analogs (for example U.S. Patents Nos 5,516,785; 5,602,257; 5,741,802 and 5,840,906).

Various patents assigned to Sibia describe the use of polycyclic fused ring compounds (U.S. Patent 5,567,710) or substituted pyridines (U.S. Patents 5,565,388; 5,594,011; 5,703,100; 5,705,512; 5,723,477 and 5,852,041) as having nicotinic properties.

Dull and his co-workers describe a variety of aryl substituted olefinic and acetylenic compounds and aza-bicyclo compounds as interacting with nicotinic receptors in U.S. Patents 5,616,707; 5,583,140; 5,597,919; 5,616,716; 726,316; 5,731,314; 5,824,692; 5,861,423; 5,885,998; 5,922,723; 5,952,339; 6,057,446; 6,100,269; 6,107,298; 6,211372; 6,218383; 6,232,316; 6,262,134; 6,274,600; 6,310,102 and 6,337,351.

A similar teaching relating to spiro-azabicyclo compounds is found in U.S. Patent 6,110,914 (Phillips).

A number of patents assigned to Abbott Laboratories describe heterocyclic ethers, particularly those containing a pyridyloxymethyl group, as having activity as ligands at neuronal nicotinic cholinergic channel receptors. Such patents include U.S. Patents 5,629,325; 5,733,912; 5,948,793; 6,127,386 and 6,133,253.

Substituted amines useful as ligands for nicotinergic receptors are described in Kruger et al's U.S. Patent 5,547,965.

Substituted pyridines are described as being of use as *inter alia* as allosteric modulators of acetyl choline receptors in U.S. Patent 6,194,581 (Cosford) and similar teaching relating to substituted aryl compounds containing ether, ester, amide, keto or thioether functionality is found in U.S. Patent 6,316,490 (Vernier et al).

5-Hydroxyindole is taught to be a nicotinic agonist in .U.S. Patent 6,277,870 (Gurley).

Certain N-(pyridinylmethyl-heterocylylylidene amines and diazocin-8-one derivatives are described as being useful in treating nicotine addiction in U.S. Patents Nos 6,020,335 (Nagel et al) and 6,235,734 (O'Neil) respectively.

Nornicotine compounds are described in U.S. Patent 5,776,957 (Crooks et al.) as having activity at nicotinic receptors.

The use of epibatidine and derivatives thereof as nicotinic cholinergic receptor agonists is described in U.S. Patents 5,817,679; 6,060,473; 6,077,846; 6,177,451 and 6,117,889.

The compounds described in the above mentioned patents may find use in the method of the present invention.

The method of the present invention is of particular use in conjunction twith the use of cholesterol-lowering drugs such as lovastatin, sinvastatin, pravastatin, and fluvastatin. The method may also be of use in conjunction with colestipol, clofibrate, gemfibrozil, fenofibrate, nicotinic acid and other hypolipidemic agents or conditions which lower cholesterol levels.

Dosages for suitable agents can be determined by standard techniques such as those set out for example in Chapter 6 (by Benjamin Calesnick) of Drill's Pharmacology in Medicine (Fourth Edition Joseph R. DiPalma ed, McGraw-Hill 1971 or in Chapter 6 (by B. E. Rodda et al) of Biopharmaceutical Statistics for Drug Development (ed. Karl E. Peace, Marcel Dekker Inc. 1988).

Dosages for currently used compounds are as follows. Galanthamine is administered as 8 to 12 mg bid, however doses of 2 to 20 mg bid may be appropriate in some cases. Donepezil is administered as 5 or 10 mg qd; doses of 2 to 20 mg qd may be used. Rivastigmine is used in doses of 1.5 to 6 mg bid; doses as low as 0.75 to 9 mg bid may be appropriate. All of these drugs require dose-escalation over time to minimize side effects of nausea, vomiting and diarrhea. Nicotine patches may be used according to common practices.

From a further aspect, the present invention provides A method for treating neuromuscular dysfunction resulting from use ofHMG-CoA reductase inhibitors by modulating nicotinic receptors by administering an effective amount of a nicotinic allosteric potentiator, nicotine, a nicotinic agonist or a mixture thereof to a patient in need of such modulation.

### Features of the Parent Application include:

1. A method for treating the effects of low LDL-cholesterol values in the brain on cognitive performance or other central nervous system functions by modulating nicotinic receptors by administering an effective amount of a nicotinic allosteric potentiator, an acetylcholinesterase inhibitor, nicotine, a nicotinic agonist or a mixture thereof to a patient in need of such modulation.
2. A method as featured in feature 1, wherein said low cholesterol values are values of less than 109 mg/dl LDL-cholesterol.
3. A method as featured in feature 1, wherein said low cholesterol values are the result of treatment with HMG-CoA reductase inhibitors.
4. A method as featured in feature 1, 2 or 3, wherein said modulation of nicotinic receptors is effected by administering an effective amount of a galanthamine or lycoramine analog to a patient in need of such modulation.
5. A method as featured in feature 4, wherein said analog of galanthamine or lycoramine is one wherein at least one of the methoxy, hydroxy or methyl groups of galanthamine or lycoramine is replaced as follows:
   the methoxy group by another alkoxy group of from one to six carbon atoms, a hydroxy group, hydrogen, an alkanoyloxy group, a benzoyloxy or substituted benzoyloxy group, a carbonate group or a carbamate group or a trialkylsilyloxy group;
   the hydroxy group by an alkoxy group of from one to six carbon atoms, hydrogen, an alkanoyloxy group, a benzoyloxy or substituted benzoyloxy group, a carbonate group or a carbamate group;
   the N-methyl group by hydrogen, alkyl, benzyl, cyclopropylmethyl group or a substituted or unsubstituted benzoyloxy group.
6. A method as featured in feature 4, wherein any alkanoyloxy, carbamate and carbonate group present contains up to ten carbon atoms.
7. A method as featured in feature 4, wherein any of said alkanoyloxy, carbamate or carbonate group comprises an alkyl or alkoxy group of from 1 to 6 carbon atoms optionally substituted by one or more halo groups.
8. A method as featured in feature 4, wherein said analog is a galanthamine analog.
9. A method as featured in feature 4, wherein said analog is a lycoramine analog.
10. A method as featured in feature 4, wherein said analog is an n-butyl carbamate.
11. A method as featured in feature 4, wherein the methoxy group of galanthamine or lycoramine is replaced by a hydrogen, hydroxy or alkoxy group of from two to six carbon atoms or an acyloxy group or a mono or dialkyl carbamate or carbonate group wherein the alkyl groups contain from 1 to 8 carbon atoms.
12. A method as featured in feature 4, wherein the hydroxy group of galanthamine or lycoramine is replaced by an alkoxy group of from one to six carbon atoms, hydrogen, an acyloxy group, a carbonate group or a carbamate group which may be a mono or dialkyl or an aryl carbamate or carbonate wherein the alkyl groups contain from 1 to 8 carbon atoms.
13. A method as featured in feature 4, wherein the compound employed is one wherein the hydroxyl group of galanthamine or lycoramine is replaced by an alkanoyl group of 2 to 7 carbon atoms, a mono or dialkyl carbamate of 1-8 carbon atoms per alkyl group, a mono or diaryl carbamate, an alkyl carbonate of one to six carbon atoms in its alkyl group or an aryl carbonate.
14. A method as featured in feature 4, wherein the compound employed is one wherein the methoxy group of galanthamine or lycoramine is replaced by an alkoxy group of two to six carbon atoms or a carbonate of from one to six carbon atoms an alkyl carbonate of one to six carbon atoms in its alkyl group or an aryl carbonate.
15. A method as featured in feature 1, 2 or 3, wherein said compound is administered in conjunction with a cholesterol-lowering drug.
16. A method for treating neuromuscular dysfunction resulting from use of HMG-CoA reductase inhibitors by modulating nicotinic receptors by administering an effective amount of a nicotinic allosteric potentiator, nicotine, a nicotinic agonist or a mixture thereof to a patient in need of such modulation.
17. Use of a nicotinic allosteric potentiator, an acetylcholinesterase inhibitor, nicotine, a nicotinic agonist or a mixture thereof for preparation of a medicament for treatment of the effects of low LDL-cholesterol values in the brain on cognitive performance or other central nervous system functions.
18. A medicine for treating the effects of low LDL-cholesterol values in the brain on cognitive performance or other central nervous system functions, which medicine comprises a nicotinic allosteric potentiator, an acetylcholnesterase inhibitor, nicotine, a nicotine agonist or a mixture thereof.

## Claims

1. Galanthamine, lycoramine, donepezil, rivastigmine or an analog of galanthamine or lycoramine wherein at least one of the methoxy, hydroxy or methyl groups of galanthamine or lycoramine is replaced as follows:
the methoxy group by another alkoxy group of from two to six carbon atoms, a hydroxy group, hydrogen, an alkanoyloxy group, a benzoyloxy or substituted benzoyloxy group, a carbonate group or a carbamate group or a trialkylsilyloxy group;
the hydroxy group by an alkoxy group of from one to six carbon atoms, hydrogen, an alkanoyloxy group, a benzoyloxy or substituted benzoyloxy group, a carbonate group or a carbamate group;
the N-methyl group by hydrogen, alkyl, benzyl, cyclopropylmethyl group or a substituted or un substituted benzoyloxy group. for use in treatment of cognitive dysfunction in persons having LDL-cholesterol values of less than 109mg/dl.

2. Galanthamine, lycoramine or an analog of galanthamine or lycoramine as specified in claim 1 for use as defined in claim 1 wherein treatment is effected by modulating nicotinic receptors.

3. Galanthamine, lycoramine or an analog of galanthamine or lycoramine as specified in claim 1 for use as defined in claim 1 or claim 2 wherein the person to be treated is not suffering from Alzheimer's disease.

4. Galanthamine, lycoramine or an analog of galanthamine or lycoramine as specified in claim 1 for use as defined in claim 1, 2 or 3 , wherein said low cholesterol values are the result of treatment with HMG-CoA reductase inhibitors.

5. An analog of galanthamine or lycoramine as specified in claim 1 for use as defined in claim 1, 2 or 3 wherein any alkanoyloxy, carbamate and carbonate group present contains up to ten carbon atoms.

6. An analog of galanthamine or lycoramine as specified in claim 1 for use as defined in claim 1, 2, 3 or 4, wherein any of said alkanoyloxy, carbamate or carbonate group comprises an alkyl or alkoxy group of from 1 to 6 carbon atoms optionally substituted by one or more halo groups.

7. A galathamine n-butyl carbamate.for use as specified in Claim 1, 2, 3 or 4.

8. A galanthamine or lycoramine analog as specified in Claim 1 for a use as specified in Claim 1, 2, 3 or 4 , wherein the methoxy group of galanthamine or lycoramine is replaced by a hydrogen, hydroxy or alkoxy group of from two to six carbon atoms or an acyloxy group or a mono or dialkyl carbamate or carbonate group wherein the alkyl groups contain from 1 to 8 carbon atoms.

9. A galanthamine or lycoramine analog as specified in Claim 1 for a use as specified in Claim 1, 2 ,3 or 4, wherein the hydroxy group of galanthamine or lycoramine is replaced by an alkoxy group of from one to six carbon atoms, hydrogen, an acyloxy group, a carbonate group or a carbamate group which may be a mono or dialkyl or an aryl carbamate or carbonate wherein the alkyl groups contain from 1 to 8 carbon atoms.

10. A galanthamine or lycoramine analog as specified in Claim 1 for a use as specified in Claim 1, 2, 3 or 4, wherein the hydroxyl group of galanthamine or Iycorarnine is replaced by an alkanoyl group of 2 to 7 carbon atoms, a mono or dialkyl carbamate of 1-8 carbon atoms per alkyl group, a mono or diaryl carbamate, an alkyl carbonate of one to six carbon atoms in its alkyl group or an aryl carbonate.

11. A galanthamine or lycoramine analog as specified in Claim 1 for a use as specified in Claim 1, 2, 3 or 4 wherein .the compound employed is one wherein the methoxy group of galanthamine or lycoramine is replaced by an alkoxy group of two to six carbon atoms or a carbonate of from one to six carbon atoms an alkyl carbonate of one to six carbon atoms in its alkyl group or an aryl carbonate.
